# EUROPÄISCHE PATENTANMELDUNG

(11) **EP 4 572 336 A1**
(43) Veröffentlichungstag der Anmeldung: **18.06.2025**
(21) Anmeldenummer: 24213901.2
(22) Anmeldetag: 19.11.2024
(51) Int. Cl.: H04R 25/00, H04R 1/10

(54) **HÖRINSTRUMENT UND VERFAHREN ZUM BETRIEB EINES SOLCHEN**

(30) Priorität: 12.12.2023 DE 102023212514
(71) Anmelder: Sivantos Pte. Ltd., Singapore 539775 (SG)
(72) Erfinder: HUSUNG, Kunibert, 91058 Erlangen (DE)
(74) Vertreter: FDST Patentanwälte

(57) **Zusammenfassung**

Es wird ein Hörinstrument (4) angegeben, mit einem in einer vorgesehenen Trageposition hinter dem Ohr getragenen Gehäuse (8), einem in den Gehörgang eines Nutzers einzusetzenden Ohrstück (10) und einem flexiblen Verbindungsstück (14), das das Gehäuse (8) mit dem Ohrstück (10) verbindet. Das Hörinstrument (4) umfasst weiterhin einen Ausgangswandler (12) zur Umwandlung eines Ausgangs-Audiosignals (O) in ein an den Nutzer auszugebendes Schallsignal sowie einen kapazitiven Sensor (26), der eine Steuer- und Auswerteschaltung (28) sowie zwei damit elektrisch verbundene Sensorelektroden (30,32) aufweist. Eine der Sensorelektroden (30) ist dabei in dem Gehäuse (8) angeordnet, während die andere Sensorelektrode (32) in dem Verbindungsstück (14) und/oder dem Ohrstück (10) angeordnet ist.

## Beschreibung

Die Erfindung bezieht sich auf ein Hörinstrument mit einem in einer vorgesehenen Trageposition hinter dem Ohr eines Nutzers getragenen Gehäuse, einem in den Gehörgang eines Ohres einzusetzenden Ohrstück und einem flexiblen Verbindungsstück. Die Erfindung bezieht sich weiterhin auf Verfahren zum Betrieb eines solchen Hörinstruments.

Als Hörinstrument wird allgemein ein elektronisches Gerät bezeichnet, das das Hörvermögen einer das Hörinstrument tragenden Person (die nachfolgend als "Träger" oder "Nutzer" bezeichnet ist) unterstützt. Insbesondere bezieht sich die Erfindung auf Hörinstrumente, die dazu eingerichtet sind, einen Hörverlust eines hörgeschädigten Nutzers ganz oder teilweise zu kompensieren. Ein solches Hörinstrument wird auch als "Hörgerät" bezeichnet. Daneben gibt es Hörinstrumente, die das Hörvermögen von normalhörenden Nutzern schützen oder verbessern, zum Beispiel in komplexen Hörsituationen ein verbessertes Sprachverständnis ermöglichen. Zu den Hörinstrumenten zählen ferner auch (im oder an dem Ohr getragene) drahtlose Kopfhörer, insbesondere sogenannte Ear Plugs und Headsets.

Hörinstrumente im Allgemeinen, und Hörgeräte im Speziellen, sind meist dazu ausgebildet, am Kopf und hier insbesondere in oder an einem Ohr des Nutzers getragen zu werden, insbesondere als Hinter-dem-Ohr-Geräte (nach dem englischen Begriff "behind the ear" auch als BTE-Geräte bezeichnet) oder In-dem-Ohr-Geräte (nach dem englischen Begriff "in the ear" auch als ITE-Geräte bezeichnet). Im Hinblick auf ihre interne Struktur weisen Hörinstrumente regelmäßig mindestens einen (akusto-elektrischen) Eingangswandler, eine Signalverarbeitungseinheit (Signalprozessor) und einen Ausgangswandler auf. Im Betrieb des Hörinstruments nimmt der oder jeder Eingangswandler einen Luftschall aus der Umgebung des Hörinstruments auf und wandelt diesen Luftschall in ein Eingangs-Audiosignal (d. h. ein elektrisches Signal, das eine Information über den Umgebungsschall transportiert) um. In der Signalverarbeitungseinheit wird das oder jedes Eingangs-Audiosignal verarbeitet (d. h. hinsichtlich seiner Schallinformation modifiziert), um das Hörvermögen des Nutzers zu unterstützen, insbesondere um einen Hörverlust des Nutzers auszugleichen. Die Signalverarbeitungseinheit gibt ein entsprechend verarbeitetes Audiosignal an den Ausgangswandler aus. In modernen Hörinstrumenten umfasst die Signalverarbeitung zusätzlich oder alternativ zu einer frequenzabhängigen Verstärkung des Eingangs-Audiosignals regelmäßig eine Vielzahl von weiteren Funktionen, z.B. Beamforming (d.h. richtungsabhängige Dämpfung), aktive Geräuschunterdrückung, Windgeräuschunterdrückung. Rückkopplungsdämpfung, binaurale Verarbeitung zur Unterstützung des räumlichen Hörens, dynamische und/oder spektrale Kompression, etc.

In den meisten Fällen ist der Ausgangswandler als elektro-akustischer Wandler ausgebildet, der das (elektrische) Ausgangs-Audiosignal wieder in einen Luftschall umwandelt, wobei dieser - gegenüber dem Umgebungsschall modifizierte - Luftschall in den Gehörgang des Nutzers abgegeben wird. Bei einem hinter dem Ohr getragenen Hörinstrument ist der auch als "Hörer" ("Receiver") bezeichnete Ausgangswandler meist außerhalb des Ohrs in einem Gehäuse des Hörinstruments integriert. Der von dem Ausgangswandler ausgegebene Schall wird in diesem Fall mittels eines Schallschlauchs in den Gehörgang des Nutzers geleitet. Alternativ hierzu kann der Ausgangswandler auch in dem Gehörgang, und somit außerhalb des hinter dem Ohr getragenen Gehäuses, angeordnet sein. Solche BTE-Geräte werden (nach dem englischen Begriff "receiver in canal") auch als RIC-Geräte bezeichnet. Im Ohr getragene Hörinstrumente, die so klein dimensioniert sind, dass sie nach außen über den Gehörgang nicht hinausstehen, werden (nach dem englischen Begriff "completely in canal") auch als CIC-Geräte bezeichnet.

In weiteren Bauformen kann der Ausgangswandler auch als elektro-mechanischer Wandler ausgebildet sein, der das Ausgangs-Audiosignal in Körperschall (Vibrationen) umwandelt, wobei dieser Körperschall zum Beispiel in den Schädelknochen des Nutzers abgegeben wird.

Als "Hörsystem" wird allgemein eine Anordnung von Geräten und ggf. sonstigen Strukturen bezeichnet, die Funktionen zum Betrieb eines Hörinstruments bereitstellen. Im einfachsten Fall besteht das Hörsystem nur aus dem Hörinstrument selbst. In der Regel umfasst das Hörsystem aber zusätzlich zu dem Hörinstrument mindestens ein Peripheriegerät, das im Betrieb des Hörinstruments mit diesem zusammenwirkt, z.B. ein weiteres Hörinstrument für das andere Ohr des Nutzers, eine Fernbedienung, ein externes Mikrofon, ein Programmiergerät für das Hörinstrument oder ein Ladegerät. Bei modernen Hörsystemen ist des Weiteren zusätzlich zu dem Hörinstrument oft eine Software-Applikation vorgesehen, die auf einem (insbesondere mobilen) Computer, z.B. einem Smartphone oder einem Tablet-Computer, installierbar ist und Funktionen zum Betrieb des Hörinstruments (z.B. Fernsteuerung, Programmierung, Firmware-Updates, Datensicherung, komplexe Signalverarbeitung und/oder Internet-Verbindung) beisteuert. Der Computer, auf dem diese (nachfolgend als "Hör-App" bezeichnete) Software-Applikation installiert ist, stellt zwar im Betrieb des Hörinstruments ebenfalls ein Peripheriegerät des Hörinstruments dar, das mit dem Hörinstrument datenübertragungstechnisch verbunden ist. Der Computer gehört aber in der Regel selbst nicht zu dem Hörsystem, insofern als er unabhängig von den Komponenten des Hörsystems hergestellt und vertrieben wird und für eine Vielzahl von weiteren, nicht mit dem Hörsystem verbundenen Anwendungen verwendbar ist. Der Computer (also insbesondere das Smartphone oder der Tablet-Computer des Nutzers) wird vielmehr von der Hör-App nur als externe Ressource für Rechenleistung, Speicherplatz und Kommunikationsdienste verwendet.

Die ordnungsgemäße Funktion eines Hörinstruments und damit der Nutzen, den das Hörinstrument seinem Träger im täglichen Betrieb bringt, hängt entscheidend von dem Sitz des Gehäuses und des Ohrstücks am bzw. im Ohr des Trägers ab. Mit anderen Worten kann das Hörinstrument die ihm zugedachte Aufgabe nur dann zufriedenstellend erfüllen, wenn das Gehäuse und das Ohrstück in der jeweils vorgesehenen Trageposition hinter bzw. in dem Ohr angeordnet sind.

Das liegt einerseits daran, dass die Signalverarbeitung des Hörinstruments auf eine bestimmte akustische Situation am Ort des oder jedes Mikrofons des Hörinstruments abgestimmt ist, z.B. auf eine bestimmte Ausrichtung des (jeweiligen) Mikrofons bezüglich des Kopfes und an eine bestimmte akustische Abschattung des Mikrofons durch das Ohr. Wenn das Gehäuse des Hörinstruments abweichend von der vorgesehenen Trageposition hinter dem Ohr getragen wird, so ändert sich hierdurch auch die akustische Situation, der das Mikrofon ausgesetzt ist, und damit - bei gegebenem Umgebungsgeräusch - auch das von dem Mikrofon aufgenommene Eingangs-Audiosignal. Die auf eine andere akustische Situation abgestimmte Signalverarbeitung kann das von dem fehlpositionierten Mikrofon aufgenommene Eingangs-Audiosignal nicht mehr optimal verarbeiten. Dies kann sich beispielsweise darin äußern, dass durch fehljustiertes Beamforming das von dem Nutzer gewünschte Nutzsignal (z.B. die Stimme eines Konversationspartners) anstelle eines unerwünschten Hintergrundgeräuschs gedämpft wird, dass aktive Geräuschunterdrückung nicht mehr nicht in zufriedenstellendem Maß funktioniert, etc.

Als unerwünschter Effekt einer Fehlpositionierung des Ohrstücks kann eine verringerte akustische Abdichtung des Gehörgangs durch das Ohrstück auftreten, was zu unerwünschten Nebeneffekten führen kann, z.B. einem erhöhten Störgeräuschlevel oder dem Auftreten von akustischen Rückkopplungen. Bei einem BTE-Gerät kann auch eine Fehljustierung des Gehäuses solche Effekte verursachen, beispielsweise dann, wenn das Verbindungstück infolge der Fehlpositionierung des Gehäuse unter mechanischer Spannung steht oder zu stark durchgebogen wird. In diesem Fall kann das Verbindungsstück eine mechanische Belastung auf das Ohrstück ausüben, die das Ohrstück allmählich ganz oder teilweise aus dem Ohr zieht oder in sonstiger Weise aus dem korrekten Sitz im Gehörgang auslenkt.

Der Erfindung liegt vor diesem Hintergrund die Aufgabe zugrunde, eine verbesserte Prüfung des ordnungsgemäßen Sitzes eines Hörinstruments zu ermöglichen.

Bezüglich eines Hörinstruments wird diese Aufgabe erfindungsgemäß gelöst durch die Merkmale des Anspruchs 1. Bezüglich eines Verfahrens zum Betrieb eines Hörinstruments wird die obige Aufgabe erfindungsgemäß gelöst durch die Merkmale des Anspruchs 6. Vorteilhafte und teils für sich gesehen erfinderische Ausgestaltungen und Weiterentwicklungen der Erfindung sind in den Unteransprüchen und der nachfolgenden Beschreibung dargelegt.

Die Erfindung geht aus von einem Hörinstrument mit einem Gehäuse, das in einer vorgesehenen Trageposition hinter dem Ohr eines Nutzers zu tragen ist. Das Hörinstrument umfasst zusätzlich ein in dem Ohr des Nutzers zu platzierendes Ohrstück, ein flexibles Verbindungsteil, das das Gehäuse und das Ohrstück verbindet und einen Ausgangswandler zur Umwandlung eines (elektrischen) Ausgangs-Audiosignals in ein an den Nutzer auszugebendes Schallsignal. Der Ausgangswandler ist vorzugsweise durch einen elektro-akustischen Wandler, also einen Lautsprecher ("Hörer") gebildet. Bei dem Hörinstrument handelt es sich also um ein BTE-Gerät, und zwar wahlweise um ein klassisches Hörinstrument mit einem in dem Gehäuse angeordneten Ausgangswandler oder um ein RIC-Gerät, bei dem der Ausgangswandler in dem Ohrstück angeordnet ist. In dem ersteren Fall ist das Verbindungsstück durch einen Schallschlauch gebildet, der den von dem Ausgangswandler produzierten Schall dem Ohrstück zuleitet. In dem letzteren Fall umfasst das Verbindungsstück ein elektrisches Verbindungskabel, durch das das Ausgangs-Audiosignal dem im Ohrstück angeordneten Ausgangswandler zugeleitet wird.

In allen vorstehend beschriebenen Ausführungsformen umfasst das Hörinstrument weiterhin einen kapazitiven Sensor, der eine Steuer- und Auswerteschaltung (nachfolgend auch als "Sensorsteuerung" oder "sensor controller" bezeichnet) sowie (mindestens) zwei damit elektrisch verbundene Sensorelektroden umfasst. Erfindungsgemäß ist eine der beiden Sensorelektroden in dem Gehäuse angeordnet. Die andere Sensorelektrode ist dagegen in dem Verbindungsstück und/oder dem Ohrstück angeordnet.

Mit dem kapazitiven Sensor können allgemein dielektrisch wirksame oder elektrisch leitfähige Strukturen in der Umgebung des Sensors detektiert werden, nämlich insbesondere Körperstrukturen wie z.B. der Kopf und das Ohr des Nutzers. Hierbei wird ausgenutzt, dass das von dem kapazitiven Sensor ausgegebene (kapazitive) Sensorsignal aufgrund der Vielfalt der umgebenden Körperstrukturen des Nutzers in charakteristischer Weise von dem Sitz des Gehäuses und des Verbindungsteils sowie ggf. des Ohrstücks an bzw. in dem Ohr abhängt. Mit anderen Worten ändert sich das von dem kapazitiven Sensor ausgegebene Sensorsignal in charakteristischer Weise, wenn der Nutzer das Hörinstrument (insbesondere das Gehäuse desselben) relativ zu dem Ohr verschiebt. Erkannterweise kann der korrekte Sitz des Hörinstruments in der vorgesehenen Trageposition anhand von dieser Abhängigkeit des kapazitiven Sensorsignals erkannt werden. Die Verteilung der zwei Sensorelektroden des kapazitiven Sensors auf das Gehäuse einerseits und das Verbindungsteil und/oder das Ohrstück andererseits hat sich hierbei als besonders als besonders vorteilhaft herausgestellt, da mit dieser Anordnung der Sensorelektroden eine besonders präzise und effektive Erkennung einer etwaigen Fehlpositionierung des Hörinstruments möglich ist. Insbesondere lässt sich hiermit effektiv feststellen, ob das Verbindungsstück bei eingesetztem Hörinstrument wie vorgesehen dicht an dem Ohr entlangläuft, woraus wiederum auf die Lage des Gehäuses und des Ohrstücks geschlossen werden kann.

Die Steuer- und Auswerteschaltung (Sensorsteuerung) des kapazitiven Sensors ist vorzugsweise dazu eingerichtet, an mindestens eine der Sensorelektroden eine elektrische Wechselspannung (auch als "(kapazitive) Sensorspannung" bezeichnet) anzulegen und ein unter Wirkung dieser Wechselspannung erzeugtes Antwortsignal zu messen, das für eine der Sensorelektrode zugeordnete elektrische Kapazität charakteristisch ist. Die Sensorsteuerung ist vorzugsweise als Mikrocontroller ausgebildet. Die Funktionalität der Sensorsteuerung ist in diesem Fall als Software implementiert. Die Sensorsteuerung kann aber im Rahmen der Erfindung auch als nicht programmierbare (analoge oder digitale) elektrische Schaltung ausgebildet sein. In beiden Fällen kann die Sensorsteuerung im Rahmen der Erfindung wahlweise als eigenständiges Bauteil (z.B. als separater integrierter Schaltkreis) ausgebildet oder zusammen mit anderen Funktionen in einer größeren Baueinheit integriert sein.

Um etwaige Störungen des von dem Ausgangswandler ausgegebenen Schallsignals durch den kapazitiven Sensor auszuschließen, ist die Sensorsteuerung vorzugsweise dazu eingerichtet, die Sensorspannung als Wechselspannung mit einer Frequenz auszugeben, die das hörbare Frequenzspektrum übersteigt, insbesondere 20 kHz nicht unterschreitet. In bevorzugter Ausführung liegt die Frequenz der Sensorspannung zwischen 20 kHz und 10 MHz, insbesondere zwischen 20 kHz und 100 kHz, z.B. bei 60 kHz.

Der kapazitive Sensor kann im Rahmen der Erfindung grundsätzlich auf einem von zwei an sich herkömmlichen Funktionsprinzipien beruhen.

Gemäß einem ersten Funktionsprinzip, das z.B. als "Ein-Elektrodenmessung" oder "Selbst-Kapazitäts-Messung" (Self Capacitance Sensing) bezeichnet wird, misst die Sensorsteuerung das Antwortsignal an derselben Sensorelektrode, an der sie die Sensorspannung anlegt. Die Sensorelektroden des kapazitiven Sensors werden dabei von der Sensorsteuerung unabhängig voneinander mit der Sensorspannung angesteuert. Als kapazitätsabhängiges Antwortsignal misst die Sensorsteuerung dabei an jeder Sensorelektrode beispielsweise den unter Wirkung der Sensorspannung auf diese Sensorelektrode fließenden Strom oder die Frequenz der Sensorspannung (wobei ausgenutzt wird, dass die jeweilige Sensorelektrode Teil eines Schwingkreises mit einer kapazitätsabhängig variierenden Resonanz ist). In allen Fällen ist das Antwortsignal hierbei charakteristisch für die (elektrische) Kapazität einer jeden Sensorelektrode gegenüber einem externen Massepotential. Als Gegenelektrode des Kondensators, dessen Kapazität der Sensor misst, wirkt bei der "Ein-Elektrodenmessung" somit das externe Massenpotential, das hier z.B. durch den Körper des Nutzers gebildet wird.

Gemäß einem zweiten Funktionsprinzip, das z.B. als "Zwei-Elektrodenmessung" , "Sender-Empfänger-Prinzip" oder "Relativ-Kapazitäts-Messung" (Mutual Capacitance Sensing) bezeichnet wird, und legt die Sensorsteuerung die Sensorspannung an einer ersten Sensorelektrode (Sendeelektrode) an und misst das Antwortsignal an der anderen Sensorelektrode (Empfangselektrode). Als kapazitätsabhängiges Antwortsignal misst die Sensorsteuerung in diesem Fall z.B. den unter Wirkung der Sensorspannung über ein elektrisches Feld in der Empfangselektrode erzeugten Strom. Das Antwortsignal hierbei charakteristisch für die (elektrische) Kapazität des Kondensators, der durch die beiden Sensorelektroden gebildet wird. Bei der "Zwei-Elektrodenmessung" wirkt der Körper des Nutzers als Störpotential, das die Kapazität des durch die beiden Sensorelektroden gebildeten Kondensators verändert und hierüber detektiert wird.

Der kapazitive Sensor des erfindungsgemäßen Hörinstruments ist bevorzugt als "Relativ-Kapazitäts-Sensor" (Mutual Capacitance Sensor) ausgebildet, da in dieser Ausführung die Sensorsteuerung schaltungstechnisch besonders einfach realisiert werden kann; in einer besonders einfachen Ausführung umfasst die Sensorsteuerung in diesem Sinne eine einkanalige Auswerteschaltung, weist also lediglich einen einzigen Sensoreingang zur Messung eines einzigen Antwortsignals auf.

Wie vorstehend erwähnt, ist das Hörinstrument in bevorzugten Ausführungsformen als RIC-Gerät mit einem in dem Ohrstück angeordneten Ausgangswandler (insbesondere einem elektro-akustischen Ausgangswandler) ausgebildet. Das in dem Verbindungstück enthaltene elektrische Verbindungskabel, über das das Ausgangs-Audiosignal dem Ausgangswandler zugeführt wird, ist hierbei vorzugsweise als eine der Sensorelektroden des kapazitiven Sensors genutzt. Im Rahmen der Erfindung können hierbei die elektrischen Signalleitungen des Verbindungskabels, über die das Ausgangs-Audiosignal dem Ausgangswandler zugeführt ist, als Sensorelektrode des kapazitiven Sensors mitgenutzt werden. Alternativ hierzu enthält das Verbindungskabel zusätzlich zu den Signalleitungen einen separaten elektrischen Leiter, der (insbesondere ausschließlich) als Sensorelektrode genutzt wird. Zusätzlich oder alternativ zu der Verwendung des Verbindungskabels als Sensorelektrode wird der Ausgangswandler (insbesondere ein metallisches Gehäuse des Ausgangswandlers) als eine der Sensorelektroden des kapazitiven Sensors genutzt. In einer zweckmäßiger Ausgestaltung sind das Verbindungskabel und der Ausgangswandler zur Bildung einer einzigen Sensorelektrode miteinander kurzgeschlossen; insbesondere wird die Sensorelektrode hierbei durch einen Masseleiter oder einen Schirmleiter des Verbindungskabels gebildet, der mit dem metallischen Gehäuse des Ausgangswandlers kurzgeschlossen ist. Im Rahmen der Erfindung ist alternativ aber auch denkbar, dass das Verbindungskabel und der Ausgangswandler im Rahmen des kapazitiven Sensors als verschiedene, insbesondere voneinander unabhängig angesteuerte Sensorelektroden genutzt sind.

In weiteren Ausführungsformen der Erfindung ist das Hörinstrument - wie ebenfalls vorstehend erwähnt - als klassisches BTE-Gerät ausgebildet, wobei der Ausgangswandler in dem Gehäuse angeordnet ist. Das Verbindungsstück ist hierbei als hohler Schallschlauch ausgebildet, der dazu dient, das von dem - in diesem Fall stets elektro-akustisch ausgebildeten - Ausgangswandler erzeugte Schallsignal zu dem Ohrstück zu leiten. Eine der Sensorelektroden des kapazitiven Sensors ist in diesem Schallschlauch angeordnet, z.B. in Form eines in dem Schallschlauch geführten oder in die Wand des Schallschlauchs eingebetteten Drahts oder Drahtnetzes oder Drahtgeflechts oder in Form einer auf der Innenwand des Schallschlauche aufgebrachten, elektrisch leitfähigen Folie oder Beschichtung.

Ein weitere Verkörperung der Erfindung bezieht sich auf ein Hörsystem, das das erfindungsgemäße Hörinstrument (insbesondere in einer der vorstehend beschriebenen Varianten) sowie eine Sitzkontrolleinheit umfasst. Die Sitzkontrolleinheit ist dabei dazu eingerichtet, anhand eines von dem kapazitiven Sensor ausgegebenen Sensorsignals den Sitz des Hörinstruments an dem Ohr des Nutzers zu prüfen. Die Sitzkontrolleinheit, die wahlweise als Software-Modul oder als nicht-programmierbare elektronische Schaltung realisiert ist, nimmt diese Prüfung vorzugsweise vor, indem sie das von dem kapazitiven Sensor ausgegebene Sensorsignal mit einem hinterlegten Referenzwert vergleicht. Da jeder Nutzer eine individuelle Kopf- und Ohrenform hat, wird vorzugsweise auch der Referenzwert hierbei vorzugsweise individuell für den jeweiligen Nutzer bestimmt. In einer alternativen Ausführung umfasst die Sitzkontrolleinheit ein neuronales Netzwerk, dem das Sensorsignal des kapazitiven Sensors zugeführt wird, und das insbesondere individuell auf den jeweiligen Nutzer eingelernt ist, um anhand des Sensorsignals den Sitz des Hörinstruments zu prüfen. Die Sitzkontrolleinheit ist vorzugsweise in dem Hörinstrument integriert. In diesem Fall kann das Hörsystem auch nur aus dem mit der Sitzkontrolleinheit ausgestatteten Hörinstrument selbst bestehen. Alternativ dazu ist die Sitzkontrolleinheit außerhalb des Hörinstruments, z.B. in einem Peripheriegerät oder einer Software-Applikation (Hör-App) des Hörsystems angeordnet.

Die Sitzkontrolleinheit ist weiterhin dazu eingerichtet, bei Feststellung einer Abweichung des Sitzes von der vorgesehenen Trageposition die Ausgabe einer Meldung zu veranlassen, die den Nutzer auf den abweichenden Sitz des Gehäuses hinweist. Diese Meldung wird vorzugsweise durch Ausgabe einer Textnachricht oder eines graphischen Hinweises (z.B. in Form einer schematischen Darstellung des an dem Ohr angeordneten Hörinstrumentengehäuses) auf einem Peripheriegerät angezeigt, z.B. durch die dem Hörinstrument zugeordnete Hör-App auf dem Display eines Smartphones des Nutzers. Zusätzlich oder alternativ wird die Meldung durch das Hörinstrument selbst ausgegeben, z.B. in Form eines Signaltons, einer über den Hörer des Hörinstruments ausgegebenen Sprachnachricht oder durch ein taktiles Signal (z.B. Vibration).

In einer weiteren Ausführung ist die Sitzkontrolleinheit dazu eingerichtet, anhand des von dem kapazitiven Sensor ausgegebenen kapazitiven Sensorsignals mindestens einen Signalverarbeitungsparameter des Hörinstruments einzustellen, um die Signalverarbeitung (d.h. die Funktion des Signalprozessors) des Hörinstruments an den Sitz des Gehäuses anzupassen. Insbesondere passt die Sitzkontrolleinheit hierbei die Ausrichtung eines Beamformers des Hörinstruments derart an, dass eine festgestellte Abweichung des Sitzes des Gehäuses von der vorgesehenen Trageposition kompensiert wird.

Das erfindungsgemäße Verfahren nutzt das erfindungsgemäße Hörinstrument in einer der vorstehend beschriebenen Ausführungsformen. Die vorstehenden Angaben zu Ausführungsvarianten und Bestandteilen des Hörinstruments und den damit verbundenen Effekten und Vorteilen gelten daher sinngemäß auch für entsprechende Ausführungsformen des Verfahrens, und umgekehrt.

Im Zuges des erfindungsgemäßen Verfahrens wird unter Nutzung der auf das Gehäuse und das Verbindungsteil und/oder das Ohrstück verteilten Sensorelektroden des kapazitiven Sensors des erfindungsgemäßen Hörinstruments ein kapazitives Sensorsignal als Maß für den Sitz des Gehäuses und des Verbindungsteils am Ohr des Nutzers (sowie ggf. den Sitz des Ohrstücks in dem Ohr des Nutzers) ermittelt. Bei einer Feststellung einer Abweichung des Sitzes von der vorgesehenen Trageposition des Gehäuses bzw. des Verbindungsstücks und/oder des Ohrstücks wird dabei eine auf den abweichenden Sitz des Hörinstruments hinweisende Meldung ausgegeben. Zusätzlich oder alternativ hierzu wird anhand des von dem kapazitiven Sensor ausgegebenen kapazitiven Sensorsignals mindestens ein Signalverarbeitungsparameter des Hörinstruments eingestellt, um die Signalverarbeitung des Hörinstruments an den Sitz des Hörinstruments anzupassen.

Nachfolgend werden Ausführungsbeispiele der Erfindung anhand einer Zeichnung näher erläutert. Darin zeigen:

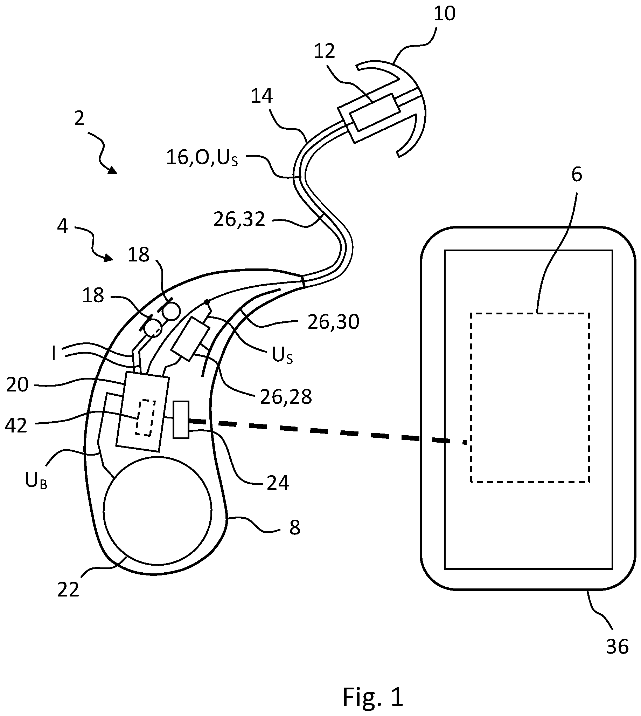

- Fig. 1: in einer schematischen Darstellung ein Hörsystem mit einem Hörinstrument und einer in einem Smartphone des Nutzers installierten Software-Applikation (Hör-App), wobei das Hörinstrument ein hinter einem Ohr eines Nutzers tragbares Gehäuse, einen in den Gehörgang des Nutzers einzusetzendes Ohrstück mit einem darin integrierten elektro-akustischen Ausgangswandler (Hörer) und ein flexibles, das Gehäuse mit dem Ohrstück mechanisch und elektrisch verbindendes Verbindungsteil aufweist, und wobei das Hörinstrument einen kapazitiven Sensor aufweist, der eine in dem Gehäuse angeordnete erste Sensorelektrode umfasst und als zweite Sensorelektrode ein in dem Verbindungsteil geführtes Verbindungskabel nutzt,
- Fig. 2: in schematischer Darstellung eine Ausführungsvariante des Hörinstruments, bei der die Sensorelektroden des kapazitiven Sensors als Sende- bzw. Empfangselektrode betrieben werden, und
- Fig. 3: in Darstellung gemäß Fig. 2 eine weitere Ausführungsvariante des Hörinstruments, bei der die Sensorelektroden des kapazitiven Sensors als unabhängig unabhängige Sensorelektroden betrieben werden.

Einander entsprechende Teile und Größen sind in allen Figuren stets mit gleichen Bezugszeichen versehen.

Fig. 1 zeigt in grob schematischer Darstellung ein Hörsystem 2, das aus einem Hörinstrument 4 und einer zugehörigen Software-Applikation (Hör-App 6) gebildet ist.

Bei dem Hörinstrument 2 handelt es sich beispielhaft um ein Hörgerät, d.h. ein zur Unterstützung des Hörvermögens eines hörgeschädigten Nutzers eingerichtetes Hörinstrument. In der hier dargestellten Ausführungsform ist das Hörinstrument 2 als RIC-Gerät ausgebildet. Es umfasst demnach ein Gehäuse 8, das bestimmungsgemäß hinter einem Ohr eines Nutzers getragen wird, sowie ein Ohrstück 10, das bestimmungsgemäß in den Gehörgang des Nutzers eingesetzt wird, wobei in dem Ohrstück 10 ein elektro-akustischer Ausgangswandler (Hörer 12) integriert ist. Das Hörinstrument 2 umfasst weiterhin ein flexibles Verbindungsstück 14, das das Gehäuse 8 mit dem Ohrstück 10 mechanisch verbindet. Im Fall des in Fig. 1 dargestellten RIC-Geräts umfasst das Verbindungsstück 14 ein elektrisches Verbindungskabel 16 für den Hörer 12.

Innerhalb eines Gehäuses 8 weist das Hörinstrument 4 folgende Komponenten auf:
- mindestens ein Mikrofon 18 (im dargestellten Beispiel zwei Mikrofone 18) als Eingangswandler,
- einen (insbesondere digitalen) Signalprozessor 20,
- eine Batterie 22,
- eine drahtlose Kommunikationseinrichtung 24 mit - nicht explizit dargestellt - einer RF-Antenne und einer damit elektrisch verbundenen Sende- und Empfangseinheit (Transceiver), sowie
- einen kapazitiven Sensor 26, der aus einer Steuer- und Auswerteschaltung (Sensorsteuerung 28) und mehreren (in dem dargestellten Ausführungsbeispiel zwei) Sensorelektroden 30 und 32 gebildet ist.

Im Normalbetrieb des Hörinstruments 4 nehmen die Mikrofone 18 jeweils einen Luftschall aus der Umgebung des Hörinstruments 4 auf. Die Mikrofone 18 wandeln den Schall in ein (Eingangs-)Audiosignal I um, d.h. in ein elektrisches Signal, das eine Information über den aufgenommenen Schall enthält. Das jeweilige Eingangs-Audiosignal I wird innerhalb des Hörinstruments 4 dem Signalprozessor 20 zugeführt, der dieses Eingangs-Audiosignal I zur Unterstützung des Hörvermögens des Nutzers modifiziert, insbesondere zur Kompensation eines Hörverlusts des Nutzers frequenzselektiv verstärkt.

Der Signalprozessor 20 gibt über (nicht näher dargestellte) Signalleitungen des in dem Gehäuse 8 und dem Verbindungsstück 14 geführten elektrischen Verbindungskabels 16 ein Ausgangs-Audiosignal O an den Hörer 12 aus. Bei dem Ausgangs-Audiosignal O handelt es sich um ein elektrisches Signal, das eine Information über den verarbeiteten und somit modifizierten Schall enthält. Der Signalprozessor 20 und alle weiteren elektrischen oder elektronischen Komponenten des Hörinstruments 4 werden aus der Batterie 22 mit einer als Betriebsspannung U_{B} bezeichneten elektrischen Gleichspannung versorgt.

Die drahtlose Kommunikationseinrichtung 24 dient zum (drahtlosen) Datenaustausch zwischen dem Hörinstrument 4 und der Hör-App 6 und/oder gegebenenfalls weiteren Komponenten des Hörsystems 2, z.B. einem (nicht dargestellten) weiteren Hörinstrument 4 für das andere Ohr des Nutzers.

Die Hör-App 6 dient insbesondere zur Fernsteuerung und Programmierung des Hörinstruments 4. Im Betrieb des Hörsystems 2 ist die Hör-App 6 lauffähig auf einem (insbesondere mobilen) Computer installiert. Im dargestellten Beispiel handelt es sich bei diesem Computer um ein Smartphone 36 des Nutzers. Der Computer, insbesondere das Smartphone 36, ist dabei selbst kein Bestandteil des Hörsystems 2, sondern wird von der Hör-App 6 nur als externe Ressource für Rechenleistung, Speicherplatz und Kommunikationsdienste verwendet. Insbesondere greift die Hör-App 6 auf eine (nicht näher dargestellte) drahtlose Kommunikationseinrichtung des Smartphones 36 zu, um Daten mit dem Hörinstrument 4 auszutauschen.

Die drahtlose Kommunikationseinrichtung 24 des Hörinstruments 4 und die drahtlose Kommunikationseinrichtung des Smartphones 36 sind dabei allgemein zum Austausch von Funksignalen (auch: Radiowellen, nämlich elektromagnetischer Strahlung mit einer Funkfrequenz von mehr als 100 MHz) ausgebildet. Vorzugsweise erfolgt die Datenübertragung zwischen dem Hörinstrument 4 und dem Smartphone 36 (und damit der Hör-App 6) auf Basis des Bluetooth-Standards, bei einer Funkfrequenz von 2,4 GHz.

Der kapazitive Sensor 26 dient zur Detektion des Sitzes des Hörinstruments 2 am Ohr des Nutzers, also zur Prüfung, ob sich das Gehäuse 8 in einer vorgesehenen Trageposition hinter dem Ohr des Nutzers oder einer davon abweichenden Lage befindet. Eine erste Sensorelektrode 30 des kapazitiven Sensors 26 ist dabei durch eine ein- oder zweidimensionale elektrisch leitfähige Struktur gebildet, die in dem Gehäuse 8, insbesondere anliegend an oder nahe zu einer Innenseite der Gehäusewand angeordnet ist. Die erste Sensorelektrode 30 ist beispielsweise durch einen Drahtleiter, ein Drahtgitter oder -geflecht, ein Metallstanzteil, eine Folie oder eine an der Innenseite der Gehäusewand aufgebrachte elektrisch leitfähige Beschichtung gebildet. Die zweite Sensorelektrode 32 ist durch den in dem Verbindungsteil 14 geführten Abschnitt des Verbindungskabels 16 gebildet, und zwar hier entweder durch einen der Signalleiter oder beide Signalleiter oder durch einen gegebenenfalls vorhandenen separaten Leiter des Verbindungskabels 16).

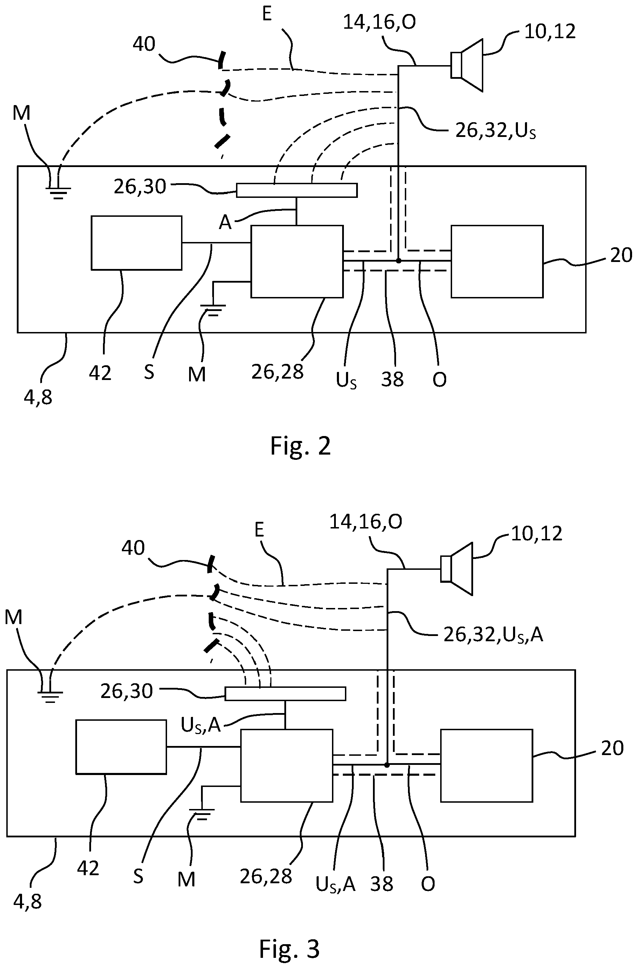

Um eine kapazitive Wechselwirkung zwischen der Sensorelektrode 30 und dem im Inneren des Gehäuses 8 geführten Abschnitt des Verbindungskabels 16 zu verhindern, ist der gehäuseinnere Abschnitt des Verbindungskabels 16 vorzugsweise von der Sensorelektrode 30 durch eine Abschirmung 38 (Fig. 2 und 3) elektrisch abgeschirmt. Ebenfalls elektrisch abgeschirmt sind vorzugsweise auch die elektrischen Zuleitungen zwischen der Sensorsteuerung 28 und den Sensorelektroden 30 und 32. Die Abschirmung 38 ist optional als potentialgesteuerter Schirm ("Driven Shield") ausgeführt.

Die Sensorsteuerung 30 steuert - wie nachstehend näher erläutert wird - mindestens eine der Sensorelektroden 30 und 32 (im Beispiel gemäß Fig. 1 das als Sensorelektrode 32 genutzte Verbindungskabel 16) mit einer als Sensorspannung Us bezeichneten elektrischen Wechselspannung an, deren Wechselspannungsfrequenz zwischen 20 kHz und 10 MHz liegt und beispielsweise 60 kHz beträgt. Die Sensorspannung Us liegt somit insbesondere spektral mit Abstand zwischen der von der Batterie 22 erzeugten Betriebsspannung U_{B} einerseits und der Funkfrequenz der drahtlosen Kommunikationseinrichtung 24 andererseits. Sie liegt ebenfalls oberhalb des hörbaren Frequenzspektrums, so dass wahrnehmbare Störungen des Ausgangs-Audiosignals O und des daraus erzeugten Schallsignals durch die in das Verbindungskabel 16 eingespeiste Sensorspannung Us ausgeschlossen sind.

Der Signalprozessor 20, der Transceiver der drahtlosen Kommunikationseinrichtung 24 und die Sensorsteuerung 28 sind jeweils wahlweise durch eine programmierbare Schaltung (z.B. einen Mikroprozessor) mit einer darin installierten Software, durch eine nicht-programmierbare Schaltung (z.B. in Form eines ASIC) oder durch eine Kombination aus mindestens einer programmierbaren Untereinheit und mindestens einer nicht-programmierbaren Untereinheit gebildet. Wie in Fig. 1 beispielhaft angedeutet, können der Signalprozessor 20, die Kommunikationseinrichtung 24 und die Sensorsteuerung 28 als jeweils separate Schaltkreise ausgebildet sein. Alternativ hierzu sind die Kommunikationseinrichtung 24 und/oder die Sensorsteuerung 28 mit dem Signalprozessor 20 und/oder mindestens einer gegebenenfalls vorhandenen weiteren Steuereinheit des Hörinstruments 4 in einem gemeinsamen Schaltkreis integriert.

In zwei alternativen Ausführungsvarianten des Hörinstruments 4 gemäß Fig. 2 und 3 werden die Sensorelektroden 30 und 32 von der Sensorsteuerung 28 unterschiedlich angesteuert.

In der Ausführung gemäß Fig. 2 werden die Sensorelektroden 30 und 32 von der Sensorsteuerung 28 als Sendeelektrode bzw. Empfangselektrode betrieben. Die Sensorsteuerung 28 legt hierbei die Sensorspannung Us an der Sensorelektrode 32 (d.h. dem Verbindungskabel 16) an. Unter Wirkung der Sensorspannung Us erzeugt die Sensorelektrode 32 in einem umgebenden Raumvolumen ein mit der Wechselspannungsfrequenz wechselndes elektrisches Feld E, das in der Sensorelektrode 30 eine Ladungsverschiebung, und damit einen elektrischen Stromfluss hervorruft. Die Sensorsteuerung 30 misst vorzugsweise die Stromstärke dieses Stromflusses an der Sensorelektrode 30 als Antwortsignal A, wobei dieses Antwortsignal A charakteristisch für die (elektrische) Kapazität des durch die Sensorelektroden 30 und 32 gebildeten Kondensators ist. Der kapazitive Sensor 26 ist somit als "Relativkapazitätssensor" (Mutual Capacitance Sensor) ausgebildet.

Nahe an dem Gehäuse 8 angeordnete (und in Fig. 4 schematisch angedeutete) Körperstrukturen 40 des Nutzers, z.B. der Kopf und das Ohr, an dem das Gehäuse 8 und das Verbindungsstück 14 getragen wird, wirken aufgrund der Wechselstromleitfähigkeit des menschlichen Körpers und der elektrischen Verbindung des Körpers mit der Sensorsteuerung 28 über Masse M als Störpotential, das die Kapazität des durch die Sensorelektroden 34a und 34b gebildeten Kondensators, und damit den Wert des gemessenen Antwortsignals A, umso mehr erniedrigt, je größer die Körperstrukturen sind, und je näher sie an den Sensorelektroden 30 und 32 angeordnet sind.

In der Ausführung gemäß Fig. 3 werden die Sensorelektroden 30 und 32 von der Sensorsteuerung 28 als separate (d.h. voneinander unabhängige) Sensorelektroden betrieben. Die Sensorsteuerung 28 legt hierbei die Sensorspannung Us an beide Sensorelektroden 30 und 32 an und misst das unter Wirkung der Sensorspannung Us erzeugte Antwortsignal A unabhängig für jede der Sensorelektroden 30 und 32; auch in diesem Fall wird vorzugsweise die Stromstärke des auf die jeweilige Sensorelektrode 30, 32 fließenden Stroms als Antwortsignal A gemessen. Das der jeweiligen Sensorelektrode 30, 32 zugeordnete Antwortsignal A ist hierbei charakteristisch für die Kapazität der jeweiligen Sensorelektroden 30 und 32 gegenüber Masse M. Der kapazitive Sensor 26 ist somit in dieser Ausführungsform als "Selbstkapazitätssensor" (Self Capacitance Sensor) ausgebildet. Die beiden Sensorelektroden 30 und 32 werden vorzugsweise gleichphasig mit der Sensorspannung Us beaufschlagt, so dass zwischen den beiden Sensorelektroden 30 und 32 selbst kein elektrisches Feld E verläuft.

Das von der jeweiligen Sensorelektrode 30, 32 unter Wirkung der Sensorspannung Us erzeugte elektrische Feld E verläuft hier zwischen der jeweiligen Sensorelektrode 30, 32 und den jeweils nächstliegenden Körperstrukturen 40 des Nutzers. Die für Wechselspannungen auf Masse M liegenden Körperstrukturen 40 des Nutzers in der Nähe des Hörinstruments 4, insbesondere also der Kopf und das angrenzende Ohr, wirken in dieser Ausführung des kapazitiven Sensors 26 als Gegenelektrode zu der jeweiligen Sensorelektrode 30, 32. Jede der Sensorelektroden 30, 32 bildet in anderen Worten zusammen mit den nächstliegenden Körperstrukturen 40 den Kondensator, dessen Kapazität an der jeweilige Sensorelektrode 30, 32 gemessen wird. Diese Kapazität und damit der Wert des gemessenen Antwortsignals A wird - anders als bei der Ausführung gemäß Fig. 2 - umso höher, je größer die Körperstrukturen 40 sind, und je näher sie an der jeweilige Sensorelektrode 30, 32 angeordnet sind.

Die Ausführungsform gemäß Fig. 2 hat gegenüber der Ausführung gemäß Fig. 3 den Vorteil, dass die Sensorsteuerung 28 hier mit einem einzigen Sensoreingang zur Messung des einen Antwortsignals A auskommt und daher besonders einfach realisierbar ist.

In der Ausführung gemäß Fig. 2 und 3 detektiert der kapazitive Sensor 26 nur den Sitz des Gehäuses 8 und des Verbindungsstücks 14 unmittelbar, da die Sensorelektroden 30 und 32 nur im Bereich dieser Teile des Hörinstruments 4 angeordnet sind. Der Sitz des Ohrstücks 10 im Ohr des Nutzers wird hier nur mittelbar überprüft, wobei angenommen wird, dass das Ohrstück 10 korrekt im Ohr des Nutzers positioniert ist, wenn die Lage des Gehäuses 8 und des Verbindungsstücks 14 mit der vorgesehenen Trageposition übereinstimmt.

In einer (nicht näher dargestellten) weiteren Ausführungsform des Hörinstruments 4 wird zusätzlich zu dem Verbindungskabel 16 auch ein metallisches Gehäuse des Hörers 12 als Sensorelektrode des kapazitiven Sensors 26 genutzt. Das Gehäuse des Hörers 12 ist hierbei vorzugsweise mit dem als Sensorelektrode 32 genutzten Leiter des Verbindungskabels 16 (insbesondere einer Schirmung des Verbindungskabels 16) kurzgeschlossen, so dass das Verbindungskabel 16 und der Hörer 12 zusammen die Sensorelektrode 32 bilden. Der kapazitive Sensor 26 detektiert in diesem Fall auch den (korrekten oder fehlerhaften) Sitz des Ohrstücks im Ohr des Nutzers unmittelbar.

In alternativer Ausführung wird der Hörer 12 (genauer das metallische Gehäuse des Hörers 12) von der Sensorsteuerung 28 zusätzlich oder alternativ zu dem Verbindungskabel 16 als separate Sensorelektrode betrieben. Mit dieser separaten Sensorelektrode wird der Sitz des Ohrstücks im Ohr des Nutzers unabhängig von dem Sitz des Gehäuses 8 und des Verbindungsstücks 14 am Ohr detektiert.

In allen vorstehend beschriebenen Ausführungsformen wird das oder jedes gemessene Antwortsignal A im Betrieb des Hörinstruments 4 in einem Verfahren zur Überprüfung des Sitzes des Hörinstruments 4 herangezogen. Zur automatischen Durchführung dieses Verfahrens umfasst das Hörsystem 2 eine Sitzkontrolleinheit 42, die dazu eingerichtet ist, anhand eines von dem kapazitiven Sensor 26 ausgegebenen Sensorsignals S den Sitz des Gehäuses 8 an dem Ohr des Nutzers zu prüfen, d.h. festzustellen, ob sich das Hörinstrument 4 (also Gehäuse 8, das Verbindungsstück 14 und ggf. das Ohrstück 10) in einer vorgesehen Trageposition am Ohr des Nutzers befindet oder ob der Sitz des Gehäuses 8, des Verbindungsstücks 14 und ggf. des Ohrstücks 10 von der vorgesehenen Trageposition abweicht.

Die Sitzkontrolleinheit 42 kann im Rahmen der Erfindung durch eine nicht-programmierbare elektronische Schaltung gebildet sein, z.B. in Form oder als Teil eines ASIC. Vorzugsweise ist die Sitzkontrolleinheit 42 aber durch ein Software-Modul gebildet, das beispielsweise in dem Signalprozessor 20 (s. Fig. 1) oder ggf. einer anderen programmierbaren Steuerschaltung des Hörinstruments 4 lauffähig installiert ist. In einer weiteren Ausführung der Erfindung ist die Sitzkontrolleinheit 42 außerhalb des Hörinstruments 4 als Teil der Hör-App 6 implementiert.

Das von der Sensorsteuerung 28 an die Sitzkontrolleinheit 42 ausgegebene Sensorsignal S enthält den (unveränderten) Wert des oder jedes Antwortsignals A oder eine hieraus abgeleitete Größe, beispielsweise die den Sensorelektroden 30, 32 (gegebenenfalls jeweils) zugeordnete Kapazität oder eine dazu invertierte oder skalierte Größe.

Um den Sitz des Hörinstruments 4 hinter dem Ohr des Nutzers zu kontrollieren, vergleicht die Sitzkontrolleinheit 42 das Sensorsignal S in einer bevorzugten Ausgestaltung des Verfahrens mit einem hinterlegten Referenzwert, der den Wert des Sensorsignals S bei Positionierung des Hörinstruments 4 (also insbesondere des Gehäuses 8 und des Verbindungsstücks 14) in der vorgesehenen Trageposition wiedergibt. Sofern die Sitzkontrolleinheit 42 eine signifikante Abweichung des Sensorsignals S von dem Referenzwert feststellt (insbesondere eine Abweichung, die einen vorgegebenen Toleranzwert übersteigt, veranlasst sie die Ausgabe einer (Warn-)Meldung an den Nutzer, die den Nutzer auf den falschen (d.h. von der vorgesehenen Trageposition abweichenden) Sitz des Hörinstruments 4 hinweist.

Da der Wert des Sensorsignals S auch von der Annäherung weiterer Körperteile an das Hörinstrument 4 beeinflusst wird, z.B. der Annäherung einer Hand oder eines Fingers, diese Störeinflüsse aber in der Regel nur von kurzer Dauer sind, ist die Sitzkontrolleinheit 42 vorzugsweise dazu ausgebildet, die Ausgabe der Warnmeldung nur dann zu veranlassen, wenn das Sensorsignal S von dem Referenzwert länger als ein vorgegebenes Zeitintervall (z.B. mehr als 2 min) dauerhaft signifikant abweicht.

Die Warnmeldung wird beispielsweise in Form
- eines über den Hörer 12 in das Ohr des Nutzers abgegebenen oder von der Hör-App 6 über einen Lautsprecher des Smartphones 36 ausgegebenen Signaltons,
- einer über den Hörer 12 in das Ohr des Nutzers abgegebenen oder von der Hör-App 6 über einen Lautsprecher des Smartphones 36 ausgegebenen Sprachnachricht,
- einer von der Hör-App 6 über das Display des Smartphones 36 angezeigten Textnachricht oder Graphik,
- oder eines über das Hörinstrument 4 oder das Smartphone 36 ausgegebenen taktilen Alarms (z.B. eines Vibrationssignals)

oder einer Kombination der vorstehend genannten Benachrichtigungsmethoden erzeugt. Der Referenzwert wird vorzugsweise bei der Anpassung des Hörinstruments 4 and den Nutzer (Fitting) individuell eingelernt, z.B. indem das Sensorsignal S erfasst und als Referenzwert abgespeichert wird, nachdem das Hörinstrument 4 von einer Fachperson in der korrekten vorgesehenen Trageposition hinter dem Ohr des Nutzers positioniert wurde.

In einer weiterentwickelten Ausführung der Erfindung ist in der Sitzkontrolleinheit 42 nicht nur ein einzelner Referenzwert für das Sensorsignal S gespeichert, sondern eine Funktion oder Kennlinie bzw. Kennwertetabelle, die zusätzlich zu dem für die vorgesehene Trageposition charakteristischen Kennwert auch Kennwerte für abweichende Positionen des Hörinstruments 4 hinter dem Ohr enthält. Die Funktion, Kennlinie oder Kennwertetabelle wird beispielsweise ebenfalls bei der Anpassung des Hörinstruments an den Nutzer ermittelt, indem das Gehäuse 8 durch eine Fachperson an verschiedenen Positionen hinter dem Ohr platziert wird, und indem in jeder Position der jeweilige Wert des Sensorsignals S zusammen mit einer Positionsangabe erfasst und abgespeichert wird. In dieser Ausführung ermittelt die Sitzkontrolleinheit 42 im Betrieb des Hörinstruments 4 verfahrensgemäß durch Vergleich des aktuellen Werts des Sensorsignals S mit der Funktion, Kennlinie oder Kennwertetabelle nicht lediglich qualitativ, ob der Sitz des Hörinstruments 4 mit der vorgesehenen Trageposition übereinstimmt. Vielmehr ermittelt die Sitzkontrolleinheit 42 hierbei quantitativ, wie stark und in welche Richtung der Sitz des Hörinstruments 4 von der vorgesehenen Trageposition abweicht.

Sofern die Sitzkontrolleinheit 42 in der vorstehend genannten Ausführungsform eine signifikante Abweichung des Sensorsignals S von dem für die vorgesehene Trageposition ermittelten Referenzwert feststellt, veranlasst sie (z.B. unter Nutzung einer der vorstehend beschriebenen Methoden) die Ausgabe einer Warnmeldung an den Nutzer, die den Nutzer auf den falschen Sitz des Hörinstruments 4 hinweist und eine Anweisung zur Korrektur des Sitzes enthält.

Alternativ stellt die Sitzkontrolleinheit 42 anhand des von dem kapazitiven Sensor 26 ausgegebenen Sensorsignals S mindestens einen Signalverarbeitungsparameter des Hörinstruments 4 ein, so dass eine Auswirkung der Fehlpositionierung des Gehäuses 8 ganz oder teilweise kompensiert wird. Beispielsweise verstellt die Sitzkontrolleinheit 42 einen Beamformer des Signalprozessors 20 des Hörinstruments 4 derart, dass eine Richtkeule des Beamformers auch bei fehlpositioniertem Gehäuse 8 genauso bezüglich des Kopfes des Nutzers ausgerichtet ist wie in der vorgesehenen Trageposition (z.B. stets senkrecht zur Longitudinalachse des Kopfes).

In einer weiter verfeinerten Ausführung der Erfindung folgt die Sitzkontrolleinheit 42 einem differenziertem Verfahrensablauf. Hierbei passt die Sitzkontrolleinheit 42 den mindestens einen Signalverarbeitungsparameter des Hörinstruments 4 nur dann - wie vorstehend beschrieben - zur Kompensation eines festgestellten Fehlsitzes des Hörinstruments 4 an, wenn die festgestellte Abweichung des Sitzes des Hörinstruments 4 von der vorgesehenen Trageposition gering ist (insbesondere einen hinterlegten Schwellwert nicht übersteigt). Andernfalls, d.h. bei einer stärkeren Fehlpositionierung des Hörinstruments 4, veranlasst die Sitzkontrolleinheit 42, wie ebenfalls vorstehend beschrieben, die Ausgabe der Warnmeldung, die den Nutzer auf den Fehlsitz des Hörinstruments 4 hinweist und eine Anweisung zur Korrektur des Sitzes enthält.

In einer weiteren Ausführung der Erfindung enthält die Sitzkontrolleinheit 42 ein individuell auf den Nutzer trainiertes neuronales Netzwerk, das das Sensorsignal S als Eingangssignal enthält und im Falle eines signifikanten Fehlsitzes des Hörinstruments 4 mindestens eine Korrekturmaßnahme veranlasst, insbesondere (wie vorstehend beschrieben) eine Anpassung mindestens eines Signalverarbeitungsparameters des Hörinstruments 4 zur Kompensation des Fehlsitzes und/oder die Ausgabe einer Warnmeldung an den Nutzer.

In weiteren Ausführungen der Erfindung handelt es sich bei dem Hörinstrument um ein klassisches BTE-Gerät mit einem hinter dem Ohr getragenen Gehäuse und einem in diesem Gehäuse angeordneten Hörer. In diesem Fall ist das Verbindungsstück 14 durch einen Schallschlauch gebildet. Als Sensorelektrode 32 wird hier beispielsweise ein in dem Schallschlauch geführter Draht genutzt. Die vorstehenden Ausführungen zur Ausführung und Anordnung des kapazitiven Sensors und zur Prüfung des Sitzes des Hörinstruments 4 an dem Ohr können auf diesen Gerätetyp einfach übertragen werden.

Die Erfindung wird an den vorstehend beschriebenen Ausführungsbeispielen besonders deutlich, ist hierauf aber keineswegs beschränkt. Vielmehr können weitere Ausführungsformen der Erfindung aus den Ansprüchen und der vorstehenden Beschreibung abgeleitet werden.

### Bezugszeichenliste

- 2: Hörsystem
- 4: Hörinstrument
- 6: Hör-App
- 8: Gehäuse
- 10: Ohrstück
- 12: Hörer
- 14: Verbindungsstück
- 16: Verbindungskabel
- 18: Mikrofon
- 20: Signalprozessor
- 22: Batterie
- 24: (drahtlose) Kommunikationseinrichtung
- 26: (kapazitiver) Sensor
- 28: Sensorsteuerung
- 30: Sensorelektrode
- 32: Sensorelektrode
- 36: Smartphone
- 38: Abschirmung
- 40: Körperstruktur
- 42: Sitzkontrolleinheit

- A: Antwortsignal
- E: (elektrisches) Feld
- I: (Eingangs-)Audiosignal
- M: Masse
- O: (Ausgangs-)Audiosignal
- S: Sensorsignal
- U_{B}: Betriebsspannung
- Us: Sensorspannung

## Patentansprüche

1. Hörinstrument (4)
- mit einem in einer vorgesehenen Trageposition hinter dem Ohr eines Nutzers getragenen Gehäuse (8),
- mit einem in den Gehörgang des Ohres einzusetzenden Ohrstück (10),
- mit einem flexiblen Verbindungsstück (14), das das Gehäuse (8) mit dem Ohrstück (10) verbindet,
- mit einem Ausgangswandler (12) zur Umwandlung eines Ausgangs- Audiosignals (O) in ein an den Nutzer auszugebendes Schallsignal und
- mit einem kapazitiven Sensor (26), der eine Steuer- und Auswerteschaltung (28) sowie zwei damit elektrisch verbundene Sensorelektroden (30,32) umfasst,
wobei eine der beiden Sensorelektroden (30) in dem Gehäuse (8) angeordnet ist, und wobei die andere Sensorelektrode (32) in dem Verbindungsstück (14) und/oder dem Ohrstück (10) angeordnet ist.

2. Hörinstrument (4) nach Anspruch 1,
wobei die Steuer- und Auswerteschaltung (28) des kapazitiven Sensors (26) dazu eingerichtet ist, an eine der Sensorelektroden (32) eine elektrische Wechselspannung (Us) anzulegen und an der anderen Sensorelektrode (30) ein unter Wirkung dieser Wechselspannung (Us) erzeugtes Antwortsignal (A) zu messen, das für eine elektrische Kapazität eines durch die Sensorelektroden (30,32) gebildeten Kondensators charakteristisch ist.

3. Hörinstrument (4) nach Anspruch 1 oder 2,
wobei der Ausgangswandler (12) in dem Ohrstück (10) angeordnet ist, wobei das Verbindungsstück (14) ein elektrisches Verbindungskabel (16) zur Zuführung des Ausgangs-Audiosignals (O) zu dem Ausgangswandler (12) umfasst, und wobei das Verbindungskabel (16) und/oder der Ausgangswandler (12) als Sensorelektrode (32) des kapazitiven Sensors (26) genutzt ist.

4. Hörinstrument (4) nach Anspruch 1 oder 2,
wobei der Ausgangswandler (12) in dem Gehäuse (8) angeordnet ist, wobei das Verbindungsstück (14) als hohler Schallschlauch zur Zuführung des von dem Ausgangswandler (12) erzeugten Schallsignals zu dem Ohrstück (10) ausgebildet ist, und wobei in dem Schallschlauch mindestens eine der Sensorelektroden (32) des kapazitiven Sensors (26) angeordnet ist.

5. Hörsystem (2) mit einem Hörinstrument (4) nach einem der Ansprüche 1 bis 4, und mit einer Sitzkontrolleinheit (42), die dazu eingerichtet ist, anhand eines von dem kapazitiven Sensor (26) ausgegebenen Sensorsignals (S) den Sitz des Gehäuses (8) und des Verbindungsstücks (14) an dem Ohr des Nutzers zu prüfen und bei einer Feststellung einer Abweichung des Sitzes von der vorgesehenen Trageposition eine auf den abweichenden Sitz hinweisende Meldung auszugeben, und/oder die dazu eingerichtet ist, anhand des von dem kapazitiven Sensor (26) ausgegebenen kapazitiven Sensorsignals (S) mindestens einen Signalverarbeitungsparameter des Hörinstruments (4) einzustellen, um die Signalverarbeitung des Hörinstruments (4) an den festgestellten Sitz anzupassen.

6. Verfahren zum Betrieb eines Hörinstruments (4) nach einem der Ansprüche 1 bis 4, wobei
- mittels des kapazitiven Sensors (26) ein kapazitives Sensorsignal (S) als Maß für den Sitz des Gehäuses (8) und des Verbindungsstücks (14) an dem Ohr des Nutzers ermittelt wird, und
- bei einer Feststellung einer Abweichung des Sitzes von der vorgesehenen Trageposition eine auf den abweichenden Sitz hinweisende Meldung ausgegeben wird, und/oder
- anhand des von dem kapazitiven Sensor (26) ausgegebenen kapazitiven Sensorsignals (S) mindestens ein Signalverarbeitungsparameter des Hörinstruments (4) eingestellt wird, um die Signalverarbeitung des Hörinstruments (4) an den festgestellten Sitz anzupassen.
